# EUROPÄISCHE PATENTSCHRIFT

(11) **EP 1 812 084 B1**
(45) Veröffentlichungstag und Bekanntmachung des Hinweises auf die Patenterteilung: **14.10.2009**
(21) Anmeldenummer: 05810370.6
(22) Anmeldetag: 17.11.2005
(51) Int. Cl.: A61L 2/26, B65B 55/10, B67C 7/00

(54) **VORRICHTUNG UND VERFAHREN ZUR LAMINAREN STERILLUFTZUFÜHRUNG WÄHREND DER STERILISATION UND/ODER DER ASEPTISCHEN BEFÜLLUNG**
PROCESS AND DEVICE FOR SUPPLYING A LAMINAR FLOW OF STERILE AIR DURING STERILISATION AND/OR ASEPTIC FILLING
DISPOSITIF ET PROCEDE D'AMENEE LAMINAIRE D'AIR STERILE PENDANT DES OPERATIONS DE STERILISATION ET/OU DE REMPLISSAGE ASEPTIQUE

(30) Priorität: 18.11.2004 DE 102004055784
(43) Veröffentlichungstag der Anmeldung: 01.08.2007
(73) Patentinhaber: SIG Technology AG, 8212 Neuhausen am Rheinfall (CH)
(72) Erfinder: BERGER, Jörg, 52428 Jülich (DE); WITTE, Jörg, 41844 Wegberg (DE); MARTEN, Detlef, 41812 Erkelenz (DE); SEICHE, Werner, 50127 Bergheim (DE)
(74) Vertreter: Cohausz & Florack
(86) Internationale Anmeldenummer: PCT/EP2005/012320
(87) Internationale Veröffentlichungsnummer: WO 2006/053746

(56) Entgegenhaltungen:
- WO-A-03/051759
- DE-A1- 4 031 472
- DE-A1- 19 945 500
- US-A- 5 064 614

## Beschreibung

Die Erfindung betrifft eine Vorrichtung und ein Verfahren zum sterilisieren und/oder aseptischen Befüllen aufrecht stehender, oben offener und kontinuierlich hintereinander transportierter Behälter, insbesondere Karton/Kunststoffverbundpackungen zur Aufnahme von Getränken oder Lebensmitteln, mit einer eine Vielzahl hintereinander angeordnete Aufnahmeplätze aufweisenden Transportvorrichtung, einer Mehrzahl von oberhalb der Behälter angeordneten Zufuhrelementen, insbesondere Sterilisierdüsen und/oder Füllauslässen und wenigstens einer Absaugvorrichtung zum Entfernen der überschüssigen unsterilen und/oder sterilen Luft, wobei oberhalb der Behälter (P) Sterilluftverteilelemente zum Gleichrichten der Sterilluftströmung angeordnet sind.

Aus der Praxis sind eine Vielzahl unterschiedlicher sog. 'Füllmaschinen' bekannt, in denen kontinuierlich zugeführte Behälter wie beispielsweise Karton/Kunststoffverbundpackungen mit einer bestimmten Menge eines vorgegebenen Produktes befüllt und anschließend verschlossen werden. Damit das so abgefüllte Produkt den Haltbarkeitsanforderungen genügt, werden die Behälter vor dem Befüllen gereinigt bzw. bei aseptischer Abfüllung sogar sterilisiert, um die im Inneren der noch leeren Behälter befindlichen Keime zuverlässig abzutöten.

Dazu ist es bekannt, dass die Füllmaschinen neben der eigentlichen Fülleinheit eine dieser vorgeschaltete Sterilisiereinheit aufweisen, wobei die hintereinander transportierten und oben offenen Behälter sowohl unterhalb der Sterilisiereinheit als auch unter den Füllauslässen vorbeibewegt werden. Dabei muss die Anzahl der hintereinander angeordneten Sterilisierdüsen bzw. Füllauslässe und die darüber zugeführte Menge eines Sterilisationsmittels bzw. Produktes auf die Behältergröße, Transportgeschwindigkeit etc. abgestimmt werden. Damit nach dem Sterilisieren des Behälterinneren und vor dem Verschließen der Behälter keine Keime in die Behälter gelangen können, wird der Bereich unterhalb der Sterilisiereinheit und Fülleinheit möglichst bis zum Verschließen der Packungen mit Sterilluft beaufschlagt; wie dies beispielsweise aus der DE 36 07 322 A1 für einen Längsläufer und aus der DE 40 31 472 A1 für einen Rundläufer bekannt ist.

Bei einem aus der DE 199 45 500 C2 bekannten Verfahren zum Sterilisieren von Behältern in einer Füllmaschine wird Sterilluft oberhalb der Behälter derart zugeführt und unterhalb der Behälter abgesaugt, dass sich ein Luftvorhang bildet. Der Luftvorhang wird durch eine laminare Strömung gebildet, welche ihrerseits durch das Durchströmen einer Düsenplatte hervorgerufen wird.

Aus der gattungsgemäßen WO 03/051759 A1 ist eine Vorrichtung zum Sterilisieren und aseptischen Befüllen aufrecht stehender, oben offener und kontinuierlich hintereinander transportierter Behälter zur Aufnahme von Getränken bekannt, die eine Transportvorrichtung mit einer Vielzahl hintereinander angeordneter Aufnahmeplätze aufweist, mit einer Mehrzahl von oberhalb der Behälter angeordneten Zufuhrelementen, mit wenigstens einer Absaugvorrichtung zum Entfernen der überschüssigen Luft und mit oberhalb der Behälter angeordneten Luftverteilelementen zum Gleichrichten der Luftströmung.

Nachteilig bei den bekannten Vorrichtungen ist, dass die Behälter quer zur - im wesentlichen vertikalen-Sterilluftströmung bewegt werden. Obwohl versucht wird, durch in der Regel unterhalb der Behälter angeordnete Absaugvorrichtungen eine möglichst vertikale Sterilluftströmung zu erreichen, kommt es zu Beeinflussungen der Nachbarbehälter durch aufgrund der Bewegung entstehende Wirbel bzw. Wirbelablösungen, so dass im ungünstigsten Fall unsterile Luft in den zuvor sterilisierten Behälter gelangen kann.

Der Erfindung liegt daher die Aufgabe zugrunde, die eingangs genannte und zuvor näher beschriebene Vorrichtung zum Sterilisieren bzw, aseptischen Befüllen von Behältern bzw. das dazu verwendete Verfahren so auszugestalten und weiterzubilden, dass bei geringem konstruktiven Aufwand eine gegenseitige Beeinflussung der vertikalen Sterilluftströmung in Bezug auf benachbarte Behälter zuverlässig ausgeschlossen wird.

Hinsichtlich der Vorrichtung wird diese Aufgabe dadurch gelöst, dass Mittel zum Transport der Zufuhrelemente und Sterilluftverteilelemente gemeinsam mit den Behältern vorgesehen sind.

Hinsichtlich des Verfahrens besteht die Lösung der Aufgabe darin, dass die Sterilisierdüsen und/oder Füllauslässe gemeinsam mit den zu sterilisierenden Behältern bewegt werden und dass ein laminarer Sterilluftvorhang sich gleichfalls mit den Behältern bewegt.

Erfindungsgemäß wird eine stabile Vertikalströmung der Sterilluft erreicht, so dass die Entstehung von Wirbeln und wirbelablösungen durch die nicht mehr stattfindende Relativbewegung von Sterilluftdüsen und Behältern zuverlässig ausgeschlossen wird. Dies hat zur Folge, dass der Verbrauch von Sterilluft verringert werden kann.

In weiterer Ausgestaltung der Erfindung weist die Transportvorrichtung ein rotierendes System auf. Bei einer solchen Ausführung, einem sog. 'Rundläufer', werden die Behälter einem eine Mehrzahl von radial angeordneten Aufnahmeplätzen aufweisenden Transportrad zugeführt, welches erfindungsgemäß mit einer der Anzahl der Aufnahmeplätze entsprechenden Anzahl von Zufuhrelementen zur Zufuhr von Sterilisationsmittel bzw. von einem abzufüllenden Produkt ausgestattet ist. Die Größe des Transportrades und damit die Anzahl der dort vorgesehenen Aufnahmeplätze richtet sich im wesentlichen nach der für die Sterilisation bzw. Abfüllung erforderlichen Zeit, welche wiederum vom Behältervolumen und der Transportgeschwindigkeit der kontinuierlich bewegten Behälter abhängig ist.

Eine weitere Lehre der Erfindung sieht vor, dass oberhalb jedes Aufnahmeplatzes der Transportvorrichtung ein eigenes zentral angeordnetes Sterilluftverteilelement angeordnet ist. Dabei kann das Sterilluftverteilelement bevorzugt abgekapselt sein, so dass eine Sterilluftverteilkammer entsteht.

Gemäß einer weiteren Lehre der Erfindung weist jede Sterilluftverteilkammer eine eigene Sterilluftzufuhr auf. Auf diese Weise gelangt Sterilluft in die Sterilluftverteilkammer und wird über den Sterilluftverteiler "vertikal gleichgerichtet", so dass im gesamten Bereich der Sterilisiereinheit bzw. Fülleinheit eine gleichgerichtete vertikale laminare Sterilluftströmung anzutreffen ist.

Unabhängig von der Ausführung als Rundläufer oder Längsläufer sieht eine weitere Lehre der Erfindung vor, dass unterhalb der Behälter ein Lochblech vorgesehen ist, und dass unterhalb des Lochbleches Öffnungen der Absaugvorrichtung angeordnet sind. Auf diese Weise wird eine möglichst gleichmäßige (laminare) Vertikalströmung erreicht, da erst unterhalb des Lochbleches eine geringe Ablenkung der abzusaugenden Luft stattfindet.

Bei der Ausgestaltung als Rundläufer kann der für die Sterilisation bzw. für das aseptische Befüllen vorgesehene Rundläufer beispielsweise komplett gekapselt ausgeführt sein, wobei am Ein- bzw. Auslass der zu- bzw. abgeführten Behälter entsprechende Schleusenvorrichtungen vorgesehen sein können.

In weiterer Ausgestaltung des erfindungsgemäßen Verfahrens ist vorgesehen, dass das Sterilisieren oder aseptische Befüllen bei einem leichten Überdruck erfolgt. Durch diese Maßnahme wird sichergestellt, dass keine Außenluft in das Innere der Vorrichtung und damit in die noch nicht verschlossenen Behälter eindringen kann.

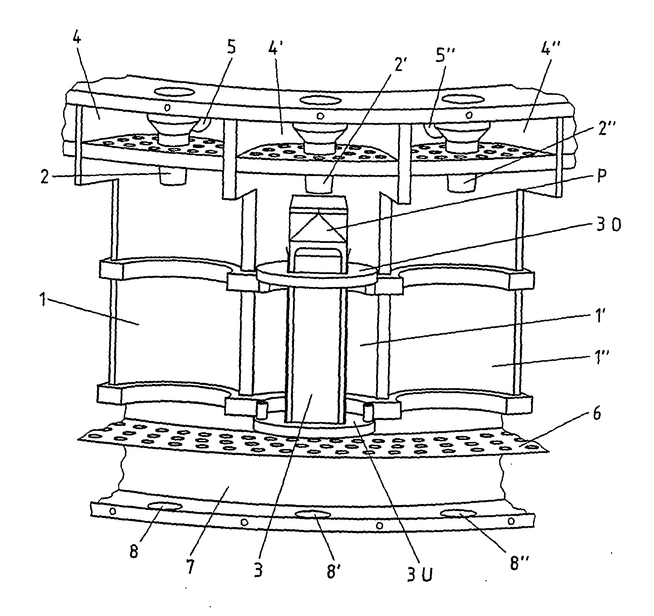

Die Erfindung wird nachfolgend anhand ein lediglich bevorzugtes Ausführungsbeispiel darstellenden Zeichnung näher erläutert. In der einzigen Figur ist in schematischer perspektivischer Ansicht ein segmentartiger Ausschnitt einer erfindungsgemäßen Vorrichtung zum Sterilisieren oder aseptischen Befüllen aufrecht stehender, oben offener und kontinuierlich hintereinander transportierter Behälter P gezeigt. Die Vorrichtung weist dabei zunächst hintereinander angeordnete Aufnahmeplätze 1, 1', 1 " auf, welche mittels einer (nicht dargestellten) Transportvorrichtung gemeinsam angetrieben werden. Im dargestellten und insoweit bevorzugten Ausführungsbeispiel sind diese am äußeren Umfang eines Transportrades angeordnet, von dem in der einzigen Figur nur ein Segment dargestellt ist.

Oberhalb der Aufnahmeplätze 1, 1', 1" sind Zufuhrelemente 2, 2', 2" angeordnet, aus denen, wie weiter unten dargestellt, ein Sterilisationsmittel, falls die Vorrichtung als Sterilisierrad arbeitet, oder aber das abzufüllende Produkt, falls die Vorrichtung als Füllrad arbeitet, in die zu sterilisierenden Behälter P geleitet wird. Die Aufnahmeplätze 1, 1', 1 " und Zufuhrelemente 2, 2', 2" werden erfindungsgemäß also gleichmäßig bewegt, wodurch eine stabile Vertikalströmung der Sterilluft erreicht wird.

Im dargestellten und insoweit bevorzugten Ausführungsbeispiel werden die Behälter P in Zellenkäfigen 3 transportiert, welche jeweils mit einem oberen Kragen 30 und einem unteren Kragen 3U versehen sind. Diese Kragen 30 bzw. 3U wirken mit dazu korrespondierenden, fest an der Vorrichtung angebrachten und nicht näher bezeichneten Stützelementen zusammen und die Zellenkäfige 3 sind dort, beispielsweise magnetisch, für die Dauer ihres Umlaufes befestigt. Zur besseren Übersicht ist in der einzigen Figur nur ein einziger Zellenkäfig 3 dargestellt, auch wenn beim Betrieb natürlich sämtliche Aufnahmeplätze 1, 1', 1 " mit Zellenkäfigen 3 versehen sind.

Oberhalb der Aufnahmeplätze 1, 1', 1" sind jeweils abgetrennte Sterilluftverteilkammern 4, 4', 4 " dargestellt, welche Einlässe 5 bzw. 5" aufweisen, da der Einlass der mittleren Sterilluftverteilkammer 4' durch das Zufuhrelement 2' verdeckt ist. Im Betrieb'sind,diese Sterilluftverteilkammern 4, 4', 4" selbstverständlich auch nach vorne verschlossen, um die durch die Einlässe 5, 5" zugeführte Sterilluft in eine vertikale Strömung "gleichrichten" zu können. Dazu weist der Boden jeder Sterilluftverteilkammer 4, 4', 4" eine Vielzahl vertikaler Öffnungen, vorzugsweise Bohrungen, auf, deren genaue Form und Größe variabel sind. Die in der Zeichnung gezeigten und nicht näher bezeichneten Bohrungen sind dabei aus Darstellungsgründen größer als die bevorzugt tatsächlich verwendeten Bohrungen.

Unterhalb der Aufnahmeplätze 1, 1', 1" ist ein waagerecht angeordnetes Lochblech 6 erkennbar, durch dessen Löcher die Luft gleichmäßig nach unten geleitet und dort durch Absaugöffnungen 8, 8', 8" abgesaugt und einer nicht dargestellten Absaugvorrichtung zugeführt wird. Auch hier sind die nicht näher bezeichneten Löcher wieder aus Gründen der Darstellung stark vergrößert gezeigt.

Nicht dargestellt ist, dass bei der Verwendung von Rundläufern das Sterilisierrad und das Füllrad nahe beieinander angeordnet sind und dass die Übergabe der Behälter P mittels einem Übergaberad erfolgt, welches zweckmäßigerweise gleichfalls gekapselt ausgeführt ist. Mit der erfindungsgemäßen Vorrichtung ist es möglich, die einmal sterilisierten Behälter während des weiteren Transports und Füllvorgangs bis zum Verschließen mit einem gleichmäßigen Vorhang von Sterilluft zu umgeben, so dass das Eindringen von Keimen ins Behälterinnere zuverlässig ausgeschlossen ist.

## Patentansprüche

1. Vorrichtung zum Sterilisieren und/oder aseptischen Befüllen aufrecht stehender, oben offener und kontinuierlich hintereinander transportierter Behälter (P), mit einer eine Vielzahl hintereinander angeordnete Aufnahmeplätze (1, 1', 1") aufweisenden Transportvorrichtung, einer Mehrzahl von oberhalb der Behälter (P) angeordneten Zufuhrelemente (2, 2', 2''), und wenigstens einer Absaugvorrichtung zum Entfernen der überschüssigen unsterilen und/oder sterilen Luft, wobei oberhalb der Behälter (P) Sterilluftverteilelemente zum Gleichrichten der Sterilluftströmung angeordnet sind,
**dadurch gekennzeichnet, dass**
Mittel zum Transport der Zufuhrelemente (2, 2', 2'') und Sterilluftverteilelemente gemeinsam mit den Behältern (P) vorgesehen sind.

2. Vorrichtung nach Anspruch 1,
**dadurch gekennzeichnet, dass**
die Transportvorrichtung ein rotierendes System mit einem die Behältern (P) aufnehmenden Transportrad aufweist.

3. Vorrichtung nach Anspruch 2,
**dadurch gekennzeichnet, dass**
oberhalb jedes Aufnahmeplatzes (1, 1', 1") der Transportvorrichtung ein eigenes Sterilluftverteilelement angeordnet ist.

4. Vorrichtung nach einem der Ansprüche 1 bis 3,
**dadurch gekennzeichnet, dass**
jedes Sterilluftverteilelement abgekapselt ist, so dass eine Sterilluftverteilkammer (4, 4', 4") entsteht.

5. Vorrichtung nach Anspruch 4,
**dadurch gekennzeichnet, dass**
jede Sterilluftverteilkammer (4, 4', 4") eine eigene Sterilluftzufuhr (5, 5") aufweist.

6. Vorrichtung nach einem der Ansprüche 1 bis 5,
**dadurch gekennzeichnet, dass**
unterhalb der Aufnahmeplätze (1, 1', 1") ein Lochblech (6) vorgesehen ist und dass Öffnungen (8) der Absaugvorrichtung unterhalb des Lochbleches (6) angeordnet sind.

7. Vorrichtung nach einem der Ansprüche 2 bis 6,
**dadurch gekennzeichnet, dass**
ein Rundläufer bis auf den Bereich des Ein- und/oder Ausschleusens der Behälter abgekapselt ausgeführt ist.

8. Verfahren zum Sterilisieren und/oder aseptischen Befüllen aufrecht stehender, oben offener und kontinuierlich hintereinander transportierter Behälter, mittels Sterilisierdüsen bzw. Füllauslässen,
**dadurch gekennzeichnet, dass**
die Sterilisierdüsen und/oder Füllauslässe gemeinsam mit den zu sterilisierenden Behältern bewegt werden und dass ein laminarer Sterilluftvorhang sich gleichfalls mit den Behältern bewegt.

9. Verfahren nach Anspruch 8,
**dadurch gekennzeichnet, dass**
das Sterilisieren bei in der Vorrichtung herrschendem Überdruck erfolgt.

## Claims

1. Device for the sterilizing and/or aseptic filling of containers (P), standing upright, open at the top and continuously conveyed one behind another, with a conveying device having a plurality of accommodation places (1, 1', 1") arranged one behind another, a plurality of delivery elements (2, 2', 2") arranged above the containers (P) and at least one suction device for removing superfluous non-sterile and/or sterile air, wherein sterile air distribution elements for directing the sterile air flow are arranged above the containers (P),
**characterised in that**
means are provided for conveying the delivery elements (2, 2', 2") and sterile air distribution elements together with the containers (P).

2. Device according to Claim 1,
**characterised in that**
the conveying device has a rotating system with a conveying wheel accommodating the containers (P).

3. Device according to Claim 2,
**characterised in that**
an individual sterile air distribution element is arranged above each accommodation place (1, 1', 1") of the conveying device.

4. Device according to any one of Claims 1 to 3,
**characterised in that**
each sterile air distribution element is encapsulated in such a way that a sterile air distribution chamber (4, 4',4") is formed.

5. Device according to Claim 4,
**characterised in that**
each sterile air distribution chamber (4, 4', 4") has its own sterile air delivery feed (5, 5").

6. Device according to any one of Claims 1 to 5,
**characterised in that**
a perforated plate (6) is provided beneath the accommodation places (1, 1', 1"), and **in that** openings (8) of the suction device are arranged beneath the perforated plate (6).

7. Device according to any one of Claims 2 to 6,
**characterised in that**
a circular runner is arranged encapsulated as far as the area of the intake and/or outlet lock arrangement of the containers.

8. Method for the sterilizing and/or aseptic filling of containers, standing upright, open at the top and continuously conveyed one behind another, by means of sterilizing nozzles and filling outlets respectively,
**characterised in that**
the sterilizing nozzles and/or filling outlets are moved together with the containers which are to be sterilized, and **in that** a laminar sterile air curtain likewise moves with the containers.

9. Method according to Claim 8,
**characterised in that**
the sterilization takes place with overpressure prevailing in the device.

## Revendications

1. Dispositif pour stériliser et/ou remplir de manière aseptique des récipients (P) debouts, ouverts sur le dessus et transportés en continu l'un derrière l'autre, avec un dispositif de transport présentant une série de postes de réception successifs (1, 1', 1"), une série d'éléments d'apport (2, 2', 2") disposé au-dessus des récipients (P), et au moins un dispositif d'extraction pour éliminer l'air non stérile et/ou stérile en excès, où au-dessus des récipients (P), des éléments de distribution d'air stérile sont agencés pour l'orientation du courant d'air stérile, **caractérisé en ce que** des moyens pour le transport des éléments d'apport (2, 2', 2") et des éléments de distribution d'air stérile en commun avec les récipients (P) sont prévus.

2. Dispositif selon la revendication 1, **caractérisé en ce que** le dispositif de transport présente un système rotatif avec une roue de transport réceptionnant les récipients (P).

3. Dispositif selon la revendication 2, **caractérisé en ce qu'**au-dessus de chaque poste de réception (1, 1', 1") du dispositif de transport, est agencé un élément propre de distribution d'air stérile.

4. Dispositif selon l'une des revendications 1 à 3, **caractérisé en ce que** chaque élément de distribution d'air stérile est isolé de sorte qu'il se forme une chambre de distribution d'air stérile (4, 4', 4").

5. Dispositif selon la revendication 4, **caractérisé en ce que** chaque chambre de distribution d'air stérile (4, 4', 4") présente un apport propre d'air stérile (5, 5").

6. Dispositif selon l'une des revendications 1 à 5, **caractérisé en ce que** sous les postes de réception (1, 1', 1"), est prévue une tôle perforée (6) et **en ce que** les ouvertures (8) du dispositif d'extraction sont agencés sous la tôle perforée (6).

7. Dispositif selon l'une des revendications 2 à 6, **caractérisé en ce qu'**une table tournante est développée de manière isolée, jusqu'au niveau de remplissage et/ou d'éclusage du récipient.

8. Procédé de stérilisation et/ou de remplissage aseptique de récipients debouts, ouverts sur le dessus et transportés en continu l'un derrière l'autre, à l'aide de buses de stérilisation et respectivement, de sorties de remplissage, **caractérisé en ce que** les buses de stérilisation et/ou sorties de remplissage sont déplaçables avec les récipients à stériliser et **en ce qu'**un rideau laminaire d'air stérile se déplace avec les récipients.

9. Procédé selon la revendication 8, **caractérisé en ce que** la stérilisation est réalisée à la surpression régnant dans le dispositif.
